Europäisches Patentamt

European Patent Office

Office européen des brevets

(11) Veröffentlichungsnummer: **0 309 863 B1**

## (12) EUROPÄISCHE PATENTSCHRIFT

(45) Veröffentlichungstag der Patentschrift: **08.04.92**

(51) Int. Cl.⁵: **A61K 37/38**, A61K 39/385

(21) Anmeldenummer: **88115371.2**

(22) Anmeldetag: **20.09.88**

(54) **Mittel und Verfahren zur Auslösung einer Immunantwort auf GnRH und Immunsterilisation von Säugern.**

(30) Priorität: **30.09.87 US 103489**

(43) Veröffentlichungstag der Anmeldung:
**05.04.89 Patentblatt 89/14**

(45) Bekanntmachung des Hinweises auf die
Patenterteilung:
**08.04.92 Patentblatt 92/15**

(84) Benannte Vertragsstaaten:
**AT BE CH DE ES FR GB IT LI NL SE**

(56) Entgegenhaltungen:
**EP-A- 0 000 764**
**EP-A- 0 111 841**
**GB-A- 2 001 077**

**J. ENDOCR., vol. 63, 1974, Seiten 399-406, GB;
H.M. FRASER et al.: "Effect of active immunization to luteinizing hormone releasing hormone on serum and pituitary gonadotrophins, testes and accessory sex organs in
the male rat"**

(73) Patentinhaber: **MOBAY CORPORATION
Mobay Road
Pittsburgh Pennsylvania 15205(US)**

(72) Erfinder: **Silversides, David W.
1532 10th Avenue Apt. 2
San Francisco, CA 94122(US)**
Erfinder: **Murphy, Bruce D.
R.D. No. 5, Saskatoon
Saskatchewan, S7H 3JH(CA)**
Erfinder: **Mapletoft, Reuben J.
409 Guelp Crescent
Sastatoon Saskatchewan, S7H 4R2(CA)**
Erfinder: **Misra, Vikram
119 Albert Avenue
Saskatoon Saskatchewan, S7N 1E6(CA)**
Erfinder: **Allen, Anne Francis
1308 14th Street East
Sastatoon Saskatchewan, S7H 0A7(CA)**

(74) Vertreter: **Schumacher, Günter, Dr. et al
c/o Bayer AG Konzernverwaltung RP Patentabteilung
W-5090 Leverkusen 1 Bayerwerk(DE)**

EP 0 309 863 B1

**Beschreibung**

Der Einsatz von Antikörpern zum Eingriff in die normale Funktion des Gonadotropin freisetzenden Hormons (GnRH, auch als LHRH oder Luteinisierungshormon freisetzendes Hormon bekannt) bei Säugern bei der Stimulierung der Hypophyse zur Freisetzung von Gonadotropinen ist wohlbekannt, wurde jedoch bisher nicht bis zu einem Stadium der praktischen Nutzung entwickelt. Ein solcher Eingriff hat zur Folge, daß der behandelte Säuger für verschiedene Zeiträume unfruchtbar gemacht wird. Die Antikörper werden von dem behandelten Säuger durch aktive Immunisierung geliefert, oder sie werden durch passive Immunisierung geliefert. Die letztere Technik ist für eine Kurzzeit-Sterilisierung von Interesse, und sie ist in der US-PS 4 676 981 beschrieben. Fremde Antikörper haben jedoch typischerweise eine ziemlich begrenzte Halbwertszeit in dem Wirts-Säuger, und danach ist eine erneute Behandlung erforderlich, sofern der Zustand der unterdrückten Fertilität aufrechterhalten werden soll.

Die aktive Immunisierung wird allgemein als der wirksamere Weg zu Langzeit-Effekten anerkannt. GnRH ist jedoch nur ein Decapeptid, so daß gewisse Maßnahmen erforderlich sind, es für das Immunsystem eines Säugers sichtbar zu machen. Obwohl einige Mischungen von GnRH mit Adjuvantien oder anderen Stoffen fähig sind, die Erzeugung geeigneter Antikörper auszulösen, ist der am weitesten verbreitete Weg der einer Kopplung des GnRH-Moleküls an ein großes Protein-Träger-Molekül. Eine gute Übersicht über diese Arbeit ist erschienen in "Active immunization against LHRH in the female", einem Kapitel von I.A. Jeffcoate und B.J. Keeling auf den Seiten 363 bis 377 von "Immunological Aspects of Reproduction in Mammals, herausgegeben von D.G. Crighton, erschienen bei Butterworth's of London 1984. Dieser Übersichtsartikel diskutiert die Verwendung verschiedener Kopplungsmittel, darunter Carbodiimid {1-Ethyl-3-(3-dimethylaminopropyl)carbodiimid}, gemischte Anhydride, Glutaraldehyd und bis-diazotiertes Benzidin. Obwohl dieser Weg der Kopplung über eine Bis-Diazotierung eine zweistufige Arbeitsweise umfaßt, wie sie in "Production and Characterization of an Antiserum to Synthetic Gonadotropin-Releasing Hormone" von Y. Koch et al. in Band 55, Nr. 3 (1973) der Biochemical and Biophysical Research Communications beschrieben ist, beruht er auf einer Anlagerung an die normal vorkommenden Seiten-Gruppen der Hystidin- oder Tyrosin-Reste.

Die Fachwelt hat auch den Wert der Verwendung heterobifunktioneller Kopplungsmittel mit Haptenen erkannt, die geeignete Seiten-Gruppen aufweisen. Ein Weg beständ im Einsatz von Kopplungsmitteln, die eine mit Sulfhydryl-Gruppen reaktionsfähige Gruppe und eine mit Amino-Gruppen reaktionsfähige Gruppe tragen, bei Haptenen, die Sulfhydryl-Gruppen tragen. In einigen Fallen hat man Haptene, die nicht solche Gruppen tragen, thioliert. Eine Arbeitsweise unter Verwendung solcher hetero-bifunktioneller Mittel ist beschrieben in "A method For Preparing beta-hCG COOH Peptide-Carrier Conjugates of Predictable Composition" von A.C.J. Lee et al., Seiten 749 bis 756 in Band 17 von Molecular Immunology, erschienen bei Pergamon Press of the U.K. 1980. Sie umfaßt eine zweistufige Arbeitsweise der Umsetzung eines Träger-Proteins, das viele Amino-Gruppen besitzt, mit dem Kopplungsmittel (wie etwa 6-Maleinimidcapronsäure-acyl-N-hydroxy-succinimid-ester oder MCS) und die anschließende Reaktion des so aktivierten Trägers mit dem Sulfhydryl tragenden Peptid.

In der Fachwelt wird jedoch keine Arbeitstechnik einer aktiven Immunisierung von Säugern gegen GnRH unter Verwendung handelsüblicher unbedenklicher Adjuvantien gelehrt. Die meisten mit den bekannten GnRH-Träger-Konjugaten durchgeführten Arbeiten benutzen Adjuvantien wie Freund's vollständiges oder unvollständiges Adjuvans, die außerhalb des Laboratoriums von begrenztem Nutzwert sind. Man nimmt an, daß das auf der Tatsache beruht, daß die Immunisierungsstärke dieser Konjugate variabel und bis zu einem gewissen Grade nicht präzise steuerbar ist. Man nimmt weiter an, daß dies wiederum an den Schwierigkeiten bei der Entwicklung einer reproduzierbaren Methode der Konjugation liegt.

Die Erfindung betrifft nun die Entwicklung einer Methode, mittels derer reproduzierbare und steuerbare Arbeitsweisen der Konjugation für GnRH-Träger-Konjugate mit Hilfe hetero-bifunktioneller Kopplungsmittel verfügbar gemacht werden.

Eine Klasse leicht herzustellten der Mittel ist entwickelt worden, die in Kombination mit praktischen Impfstoff-Adjuvantien eingesetzt werden können, um einen Säuger zu einer Immunantwort gegenüber seiner nativen GnRH zu veranlassen, um dadurch die Fertilität eines Säugers zu unterdrücken, wobei diese Mittel ein Konjugat aus einem GnRH-Analogon und einem immunstimulierenden Träger enthalten. Das Analogon ist ein GnRH-Molekül, in dem die in Position 1, 6 oder 10 natürlich vorkommende Aminosäure durch Cystein ersetzt ist. Der Träger ist ein Protein, das auf dem Gebiet der Immunchemie dafür bekannt und anerkannt ist, daß es niedermolekulare Haptene für das Immunsystem von Säugern sichtbar macht.

Säuger können für beträchtliche Zeitspannen dadurch steril gemacht werden, daß man sie mit diesen konjugierten Mitteln immunisiert. Typischerweise werden diese Mittel in Kombination mit einem anerkannten Impfstoff-Adjuvans in das Kreislaufsystem eines Säugers eingeführt.

2

Das GnRH-Analogon ist eine Modifikation des natürlich vorkommenden Dekapeptids, in dem die in Position 1, 6 oder 10 vorkommende Aminosäure durch Cystein ersetzt ist. Dem natürlichen Molekül fehlen jegliche Seitenketten, die primäre Amino-, Carbonsäure- oder Sulfhydryl-Gruppen tragen. In dieser Beziehung steht die seitenständige Carboxyl-Gruppe der einen Position der Glutaminsäure für die Konjugation nicht zur Verfügung, da sie mit der α-Amino-Gruppe dieser Aminosäure zu einem Pyrrolidon-Ring cyclisiert ist. Die Analoga der Erfindung haben nicht einfach etwas mit der Öffnung dieses Rings oder einer Synthese des natürlichen GnRH mit einer uncyclisierten Glutaminsäure zu tun. Die bevorzugte reaktionsfähige Gruppe ist die Sulfhydryl-Gruppe, da viele Träger zur Verfügung stehen, die, wenn überhaupt, nicht viele solcher reaktionsfähigen Gruppen tragen.

Die GnRH-Analoga können mit Hilfe jeder beliebigen wohlbekannten Technik für die Peptid-Synthese synthetisiert werden. Eine besonders vorteilhafte Verfahrensweise ist die automatisierte Peptid-Synthese im festen Zustand. Maschinen, die eine solche Synthese bewirken, sind erhältlich von du Pont und Cambridge Research Biochemicals of Cambridge, England.

Der Träger kann einer derjenigen sein, die auf dem Gebiet der Immunologie anerkanntermaßen Haptene für das Immunsystem von Säugern sichtbar machen. Sowohl GnRH als auch seine leichter konjugierten Analoga, auf die sich die Erfindung bezieht, werden allgemein als zu klein betrachtet, um als Immunogene zu wirken; selbst wenn Antikörper an sie gebunden werden, sind sie im allgemeinen nicht fähig, die Erzeugung solcher Antikörper hervorzurufen. In den gut vierzig Jahren seit den Pionierarbeiten von Landsteiner ist die Technik des Konjugierens kleiner Moleküle, bekannt als Haptene, mit viel größeren Träger-Molekülen zu einer gut eingeführten Methode geworden. Diese Träger sind typischerweise Proteine, vorzugsweise solche Proteine, die keine nennenswerte Anzahl von Sulfhydryl-Gruppen tragen. Geeignete Polypeptide haben vorzugsweise Molekülargewichte oberhalb von etwa 15 000 u (15 000 Dalton). Eine bevorzugte Klasse von Trägern sind diejenigen, die befähigt sind, eine immunologische Antwort hervorzurufen, wie sie durch einen Antikörper-Titer gemessen wird, der gleich demjenigen oder größer als derjenige ist, der gegen das konjugierte GnRH-Analogon gerichtet ist. Zu geeigneten Trägern zählen Schlüsselloch-Napfschnecken-(Fissurella-)Hämocyanin, Schweine-Thyroglobulin, Rinder-Serumalbumin, Pferde-gamma-Globulin, Pferde-Albumin, Ovalbumin und Tetanustoxoid.

Das zur Verknüpfung des GnRH-Analogon mit dem Träger-Molekül verwendete Konjugationsmittel kann ein beliebiges Mittel sein, das wenigstens zwei reaktionsfähige Gruppen trägt. Diese Gruppen sollten sowohl gegenüber dem Träger-Molekül als auch gegenüber dem GnRH-Analogon unter Bedingungen reaktionsfähig sein, die weder den Träger noch das GnRH-Analogon nachteilig beeinflussen. Vorzugsweise tragt dieses Mittel wenigstens zwei Gruppen, die unterschiedliche Reaktivitäten aufweisen, und besonders bevorzugt tragt dieses Mittel wenigstens zwei Gruppen, die mit unterschiedlichen Partnern reaktionsfähig sind. Besonders bevorzugt sind heterobifunktionelle Mittel, und am meisten bevorzugt sind solche Mittel, die eine Gruppe tragen, die zur Reaktion mit Sulfhydryl-Gruppen fähig ist, und eine andere Gruppe, die zur Reaktion mit einer gewöhnlichen Gruppe an anerkannten Trägern fähig ist. Zu diesen gewöhnlichen Gruppen zählen die Carbonsäure- und die Amino-Gruppe. Ein besonders geeignetes Konjugationsmittel ist ein heterobifunktionelles Vernetzungsmittel, das eine mit Sulfhydryl-Gruppen reaktionsfähige Gruppe und eine mit Amino-Gruppen reaktionsfähige Gruppe tragt. Zu geeigneten Mitteln gehören m-Maleinimido-benzoyl-N-hydroxysuccinimidester, m-Maleinimido-benzoylsulfosuccinimidester, N-Succinimidyl-(4-idoacetyl)-aminobenzoat, Succinimidyl-4-(N-maleinimidomethyl)-cyclohexan-1-carboxylat, Succinimidyl-4-(p-maleinimidophenyl)-butyrat, N-Succinimidyl-3-(2-pyridyldithio)-propionat, Sulfosuccinimidyl-(4-idoacetyl)-aminobenzoat, Sulfosuccinimidyl-4-(N-maleinimidomethyl)-cyclohexan-1-carboxylat, N-Succinimidylbromoacetat und Sulfosuccinimidyl-4-(p-maleinimidophenyl)-butyrat. Besonders bevorzugte heterobifunktionelle Vernetzungsmittel sind solche, die entweder eine Maleinimid- oder eine Succinimidylcarboxylat-Gruppe tragen, und Mittel, die beide Gruppen tragen, sind besonders bevorzugt. Ein vollständiger Bereich geeigneter Mittel ist international erhältlich durch die Pierce Chemical Company of Rockford, Illinois, und der öffentlich verteilte Allgemeine Katalog dieser Gesellschaft enthält eine Offenbarung der Struktur und der Aktivitat geeigneter Mittel.

Das GnRH-Analogon kann mit dem Träger in einem breiten Bereich von Verhältnissen konjugiert werden, der in erster Linie durch praktische Erwägungen begrenzt wird. Ein Konjugations-Verhältnis von weniger als 1:1 gibt praktisch keinen Sinn, da das unkonjugierte GnRH-Analogon immunologisch inaktiv ist; es wirkt nicht als Immunogen. Hohe Konjugations-Verhältnisse neigen dazu, die Wasserlöslichkeit des Konjugats zu gefährden. Konjugations-Verhältnisse von zwischen etwa 2 und 16 GnRH-Analogen auf $10^5$ u ($10^5$ Dalton) des Trägers werden als zweckmäßig bevorzugt, jedoch scheint die immunologische Antwort nicht wesentlich von dem Konjugations-Verhältnis beeinflußt zu werden.

Die tatsächlich erhaltenen Konjugations-Verhältnisse konnen zweckmäßig durch Einbau einer Markierung in das zu konjugierende GnRH-Analogon bestimmt werden. Der Einbau von Kohlenstoff[14] in eine der

Aminosäuren, etwa Glycin, des Analogon hat sich als vorteilhaft erwiesen. Nach Abschluß des Arbeitsganges der Konjugation läßt sich das unkonjugierte GnRH-Analogon in einfacher Weise von den Träger-Molekülen, die typischerweise große Proteine sind, durch Standard-Techniken der Säulenchromatographie trennen. Eine solche Technik, die bevorzugt wird, ist die Molekülgrößen-Fraktionierung. Typischerweise wird ein Standard zur Festlegung der für diese großen Proteine repräsentativen Elutions-Volumina oder -Zeiten benutzt. Die Kohlenstoff[14]-Aktivität dieses abgetrennten Protein-Materials kann dann zur Bestimmung des GnRH-Gehaltes der Konjugate herangezogen werden.

Eine besonders bevorzugte Konjugationstechnik umfaßt eine zweistufige Arbeitsweise unter Beteiligung eines heterobifunktionellen, zur Reaktion mit Amino-Gruppen und mit Sulfhydryl-Gruppen befähigten Konjugationsmittels und eines einen Cystein-Rest enthaltenden GnRH-Analogons. Eine besonders geeignete Klasse solcher Mittel sind diejenigen, die sowohl eine Maleinimid-Gruppe als auch eine Succinimidylcarboxylat-Gruppe tragen. Dieses Konjugationsmittel wird mit einem geeigneten Träger-Molekül umgesetzt, und das nichtumgesetzte Mittel von dem Träger durch Molekülgrößen-Fraktionierung abgetrennt, typischerweise durch Standard-Techniken der Säulenchromatographie. Dann wird das GnRH-Analogon zu dem aktivierten Träger hinzugefügt. Als besonders vorteilhaft hat sich erwiesen, das GnRH-Analoge als trocken zuzugeben. Der Wirkungsgrad der Kopplung oder Konjugation bei Durchführung einer solchen Arbeitsweise wurde zu annähernd 100 % gefunden.

Die sehr hohe Effizienz dieser Arbeitsweise der Konjugation erleichtert die Erzielung reproduzierbarer Ergebnisse. Somit kann man sich darauf verlassen, daß, sobald das entsprechend einem speziellen, präzisen Protokoll erzielte Konjugations-Verhältnis einmal feststeht, bei anschließender Wiederholung annähernd das gleiche Verhältnis erhalten wird.

Zuverlässigkeit und Wirkung der Konjugation bei Anwendung eines Cystein-Ersatzes kann dadurch verstärkt werden, daß man sicherstellt, daß nur die Sulfhydryl-Gruppe des Cysteins die Reaktionsfähigkeit des GnRH-Analogons erhöht. Wenn somit das Cystein eine der normalerweise in GnRH vorkommenden endständigen Aminosäuren ersetzt, sollte die freie Amino- oder Carboxyl-Gruppe des Cysteins blockiert werden. Wenn beispielsweise der Ersatz in der Eins-Position stattfindet, kann die freie Amino-Gruppe durch eine N-Acetyl-Gruppe derivatisiert werden, während dann, wenn der Ersatz in der Zehn-Position stattfindet, die freie Carboxyl-Gruppe durch Amidierung derivatisiert werden kann. Naturgemäß wird ein Ersatz in der 6-Position nicht von solchen Überlegungen betroffen; sowohl die Amino-Gruppe als auch die Carboxyl-Gruppe des Cysteins werden dann bei der Einbindung in das Peptid-Molekül vebraucht.

Die GnRH-Träger-Konjugate konnen zur Vervielfachung ihrer immunologischen Wirkung mit Adjuvantien kombiniert werden. Diese Konjugate sind selbst dann immunologisch aktiv, wenn sie in Kochsalz-Lösung verabreicht werden, jedoch kann, wie auch bei anderen Immunogenen, ihre Aktivität oder Effektivität bei der Stimulation der Antikörper-Produktion in Säugern durch Formulierung mit einem Adjuvans verstärkt werden. Geeignete Adjuvantien sind Substanzen, die in der Fachwelt anerkannt sind, die immunologische Antwort eines Säugers auf ein Immunogen zu verstärken, ohne eine unannehmbare nachteilige Reaktion zu verursachen. Beispielsweise ist Freund's vollständiges Adjuvans ein sehr wirksamer Verstarker, jedoch kein geeignetes Adjuvans, da mit seiner Anwendung Schmerz, Abszeß-Bildung und Fieber verbunden sind. Zu geeigneten Adjuvantien zählen Aluminium-Verbindungen, wie Alhydrogel (ein Aluminiumhydroxid-Gel), Wasser-in-Öl-Emulsionen, wie Freund's unvollständiges Adjuvans sowie mit Glycerin emulgiertes Erdnuß- oder Sesamöl, Muramyl-dipeptide, wie N-Acetyl-muramyl-L-alanyl-D-glutamin-n-butylester, in einem geeigneten Vehikel, wie Squalen, Liposomen, lipophilen quaternären Verbindungen, wie Dimethyldioctadecylammoniumbromid, Acrylsäure-Polymere, wie Carbopol (mit Allylsucrose vernetzte Acrylsäure), Monophosphoryllipid A sowie andere Stoffe, die eine Depot-Wirkung zeigen, d.h. das Immunogen allmählich an das Kreislauf-System des Säugers abgeben und annehmbar gut vertragen werden, wenn sie parentaral verabreicht werden. Geeignete vernetzte Acrylsäure-Adjuvantien werden in den US-PSS 3 919 411, 3 869 546 und 3 790 665 gelehrt. Rekonstituierte Adjuvantien auf Collagen-Basis werden in der US PS 3 639 577 gelehrt. Zu besonders interessanten Adjuvantien zählen Alhydrogel, Dimethyldioctadecylammoniumbromid, Dextransulfat, mit Allylsucrose vernetzte und mit Emulgator und Öl formulierte Acrylsäure, Liposomen, Threonyl-muramyl-Dipeptid in Squalen, Esopher, Regressin allein und in Öl, eine Kombination der Formulierung der vernetzten Acrylsäure mit Liposomen und eine Kombination von Regressin mit Alhydrogel.

Die Formulierung der GnRH-Träger-Konjugate mit den Adjuvantien steht im Einklang mit den normalen Empfehlungen für das Adjuvans. Im allgemeinen wird das Konjugat in solchen Formulierungen genau wie jedes andere Immunogen verwendet. Die Formulierung kann durch Zugabe eines weiteren Adjuvans oder eines anderen Verstärkungsmittels verstärkt werden. Eine klassische Definition eines Adjuvans ist ein Stoff, der einen Depot-Effekt besitzt. Einige der Stoffe, die im Vorstehenden als Adjuvantien erörtert wurden, ergeben nicht einen solchen verlängerten Zeitraum der Freisetzung des Immunogens und können in diesem Sinn als Verstärker bezeichnet werden. Beispielsweise verstärken Muramyl-Dipeptide die von einem Immunogen hervorgerufene Antwort, zeigen jedoch keine Depot-Wirkung. Solche Verstarker werden vorteilhaft mit Stoffen kombiniert, die einen solchen Depot-Effekt zeigen. In diesem Sinne werden die Muramyl-Dipeptide vorteilhafterweise mit Ölen wie Squalen oder mit Alhydrogel kombiniert.

Die Antikörper-Antwort ist nicht empfindlich gegenüber der präzisen verabreichten Dosis. Positive Ergebnisse wurden mit einer so niedrigen Dosis wie nur 10 $\mu$g GnRH-Träger-Konjugat erzielt. Eine signifikante Immunantwort wurde mit einer so kleinen Menge wie nur 0,9 $\mu$g oder 0,8 nmol des GnRH-Analogon erhalten, das mit einem geeignetem Träger konjugiert war. Eine geeignete Dosis bei kleineren Tieren, wie Mäusen, liegt zwischen etwa 10 und 200 $\mu$g Konjugat, während bei den größeren Tieren ein Bereich zwischen etwa 100 und 700 $\mu$g Konjugat bevorzugt wird, wobei ein Bereich zwischen etwa 200 und 600 $\mu$g Konjugat besonders bevorzugt wird.

Das Konjugat kann zur Auslösung einer Immunantwort bei einem beliebigen Säuger eingesetzt werden, bei dem es erwünscht ist, eine temporäre Sterilität zu erreichen. Vorzugsweise wird das Konjugat zur Immunosterilisierung von Schautieren, in Gesellschaft gehaltenen Haustieren und Schlachttieren angewandt. Besonders bevorzugt wird die Anwendung dieser Technik bei Pferden, Hunden, Katzen, Schafen und Rindern.

Der Weg der Verabreichung des Konjugats ist nicht kritisch. Bevorzugt wird jedoch ein solcher Weg, bei dem das Konjugat an das Kreislauf-System und folglich an das humorale Immunsystem des Säugers allmählich abgegeben wird. Dementsprechend wird die subkutane oder die intramuskuläre Injektion gegenüber einer intravenösen Injektion bevorzugt. Eine Verabreichung unter Beteiligung einer Passage durch den Magen-Darm-Trakt wird wegen des hochgradig wahrscheinlichen Abbaus des Konjugats und möglicher Schwierigkeiten bei einer Absorption in das Kreislauf-System nicht bevorzugt.

Die Vorschrift für die Verabreichung ist ebenfalls nicht kritisch. Es wird jedoch bevorzugt, die zweite und etwaige spätere Verabreichungen hinreichend zu verzögern, um eine Zunahme der Immunantwort gegenüber der aus der direkt vorangegangenen Verabreichung zu erreichen. Diese Antwort wird zweckmäßigerweise gemessen anhand des Titers der Antikörper des behandelten Tieres gegen GnRH. Dementsprechend wurde dann, wenn dieser Titer sich nach der Auffrischungs-Verabreichung nicht signifikant erhöht, während nach der ersten Verabreichung eine Immunantwort beobachtet worden war, die Auffrischungs-Dosis für eine Erzielung des vorteilhaftesten Ergebnisses wahrscheinlich zu früh verabreicht. Auch die Verabreichung einer Auffrischungs-Dosis, bevor die vorhergehende Verabreichung sich voll entwickelt hat, kann zu einem schlechteren als dem optimalen Ergebnis führen. Eine primäre Injektion mit nachfolgender Auffrischungs-Dosis etwa drei Wochen später hat sich als vorteilhaft erwiesen, und ebenso auch eine zweite Auffrischungs-Dosis nach etwa weiteren drei Wochen.

Das Verabreichungs-Volumen ist nicht kritisch und richtet sich vielmehr nach praktischen Erwägungen. Einerseits kann sich ein zu hohes Volumen als unzweckmäßig oder schwierig zu verabreichen erweisen, je nach der Größe und Friedlichkeit des behandelten Säugers. Andererseits kann ein minimales Volumen erforderlich sein, um die gewünschten Viskositäten zu erhalten; einige Formulierungen, etwa diejenigen unter Verwendung von Freund's vollständigem Adjuvans, benötigen nur minimale Verdünnungsmengen zur Erreichung einer Viskosität, die niedrig genug ist, damit die Nadel einer Injektionsspritze passiert werden kann. Für kleine Versuchstiere, wie Maus oder Ratte, ist ein Verabreichungs-Volumen um 250 $\mu$l zweckmäßig, während für größere Säuger, wie Rinder oder Schafe, ein Verabreichungs-Volumen um 1 ml typisch ist. Für Säuger mittlerer Größe, wie Katze oder Hund, hat sich ein Verabreichungs-Volumen von 1 ml bei einer Verabreichung an mehreren Stelle als geeignet erwiesen.

Die durch Verabreichung des GnRH-Träger-Konjugats provozierte immunologische Antwortreaktion kann in zweckmäßiger Weise durch den Antikörper-Titer oder physiologischen Effekt gemessen werden. Irgendeine der wohleingeführten Techniken zur Bestimmung des Spiegels der Antikörper gegen ein vorgegebenes Antigen, darunter der Radioimmunoassay (RIA) oder der enzym-verknüpfte Immunoadsorbens-Assay (ELISA) kann angewandt werden. Der Einsatz von RIA-Arbeitsweisen auf der Basis von Iod[125] hat sich als geeignet erwiesen. Der radioaktive Marker Iod kann mittels einer modifizierten Chloramin-T-Technik leicht an natürlich vorkommendes GnRH gebunden werden, und das ungebundene Iod kann durch Affinitätschromatographie entfernt werden. Dieses markierte GnRH kann in flüssigen oder festen Assays der primären Bindung oder in Assays der kompetitiven Hemmung verwendet werden. Ein brauchbarer Bezugspunkt für eine solche Analyse ist der Titer oder die Serum-Verdünnung, bei der die Zahl der Counts für radioaktives Iod 10 % der Gesamtzahl der verfügbaren Counts in dem Fall beträgt, in dem das gesamte Markierungsmittel an den Antikörper gebunden worden wäre.

Es wurde gefunden, daß wirksame Träger-Moleküle typischerweise eine stärkere Antikörper-Antwort provozieren als die GnRH-Analoga, mit denen sie konjugiert sind. Normalerweise ist der Titer des Anti-Träger-Antikörpers etwa eine Größenordnung hoher (eine Antwort wird bei einer wenigstens um eine Größenordnung höheren Verdünnung nachgewiesen) als diejenige des Anti-GnRH-Analogon-Antikörpers.

Die physiologische Wirkung kann mit Hilfe einer Anzahl von Techniken bewertet werden, darunter Histologie und Sexualhormon-Spiegel. Eine erfolgreiche Immunisierung mit dem GnRH-Analogon-Träger-Konjugat kann aufgrund eines verminderten Gewichts oder einer verminderten Größe der Testes oder Ovarien des behandelten Säugers nachweisbar sein. Eine positive Reaktion kann auch nachweisbar sein in der verminderten oder unterdrückten Funktion dieser Organe. Beispielsweise kann beim männlichen Tier die Spermatogenese in den Testikeln eines behandelten Säugers stark gehemmt oder ganz unterdrückt sein, und die Epididymis kann frei von Gameten sein, und im weiblichen Tier kann die Ovulation unterdrückt sein. Ein zusätzliches Anzeichen kann ein reduzierter Testosteron-oder Progesteron-Spiegel sein.

Die Erfindung wird durch die folgenden Beispiele näher erläutert. In den Beispielen beziehen sich sämtliche Angaben von "Teilen" und "Prozentzahlen" auf das Gewicht, sofern nichts anderes angegeben ist.

BEISPIEL 1

Herstellung eines Träger/GnRH-Analogon-Konjugats

Drei GnRH-Analoga wurden in der Weise durch Peptid-Synthese im festen Zustand hergestellt, daß sie die gleiche Struktur wie natürlich vorkommendes GnRH aufwiesen, jedoch mit der Abweichung, daß die erste, sechste oder zehnte natürlich vorkommende Aminosäure durch Cystein ersetzt war und im Fall des Ersatzes der endständigen Aminosäure die freie endständige Gruppe des Cysteins inaktiviert war. Das Analogon mit Ersatz in der Eins-Position beinhaltete notwendigerweise auch den Verlust der bei der normal vorkommenden Glutaminsäure auftretenden Pyrrolidon-Gruppe, und das Analogon mit Ersatz in der Zehn-Position besaß zu Anfang nicht die Amido-Gruppe, die normalerweise bei dem endständigen Glycin auftritt. Im ersteren Fall wurde die freie Amino-Gruppe N-acetyliert, und im letzteren Fall wurde die freie Carboxyl-Gruppe amidiert. Im Fall des Ersatzes in der Zehn-Position wurde eine Kohlenstoff[14] Markierung in das Glycin in der Sechs-Position eingebaut, und in den Fällen des Ersatzes in der Eins- und der Sechs-Position wurde diese Markierung in das Glycin in der Zehn-Position eingebaut.

Die Träger-Moleküle des Fissurella-Hämocyanins {"keyhole limpet hemocyanin" (KLH)} wurden für die Konjugation durch Aktivierung mit m-Maleinimido-benzoylsulfosuccinimidester (SMBS) vorbereitet. 10 mg Träger-Molekül wurden in 2 ml eines Konjugations-Puffers gelöst, der durch Zusammengeben von 8,77 g NaCl, 13,8 g $Na_2HPO_4$ und 800 ml destilliertem $H_2O$, Einstellen des pH auf 7,5 mit 1-normalen HCl und Einstellen des Volumens mit destilliertem $H_2O$ auf 1 Liter erhalten worden war. Man fügte 1,25 mg trockenes SMBS zu dieser Lösung hinzu, mischte leicht und ließ 1 h bei Raumtemperatur reagieren. Das überschüssige SMBS wurde von dem aktivierten Träger durch Fraktionierung auf einer Sephadex G 25 PD-10 Pharmacia-Säule abgetrennt, wobei der zwischen 3 und 5,5 ml eluierende hochmolekulare Peak gesammelt wurde.

Die Konjugation wurde bewirkt durch Zugabe dieser Lösung des aktivierten Trägers zu 1 mg trockenem GnRH-Analogon. Im Fall des Ersatzes in der Zehn-Position wurde das Analogon zuerst in 200 $\mu$l Ethanol solubilisiert und dann zu der Träger-Lösung hinzugefügt. In beiden Fällen folgte leichtes Vermischen und eine Reaktionsdauer von 2 h bei Raumtemperatur auf die Zugabe. Das Reaktionsbad wurde dann auf einer PD-10-Säule fraktioniert, und der zwischen 3 und 6 ml eluierende hochmolekulare Peak wurde gesammelt, um eine Abtrennung von etwaigem nicht-konjugierten Analogon vorzunehmen.

Der Protein-Gehalt des Konjugats wurde mittels des Coomasie-Blau-Protein-Assays bestimmt. Eine Standard-Kurve wurde aus Lösungen unterschiedlicher Stärken von Rinder-Serumalbumin (BSA) in dem Konjugations-Puffer durch Zusatz von 200 $\mu$l Biorad-Protein-Reagens zu 200 $\mu$l einer solchen Lösung, kurzes Durchwirbeln und Ablesen der optischen Dichte bei 595 nm hergestellt. Das Volumen der Konjugat-Lösung wurde auf 200 $\mu$l eingestellt und mit 200 $\mu$l Biorad-Protein-Reagens vereinigt. Der Vergleich der optischen Dichte bei 595 nm mit der Standard-Kurve ergab den Protein-Gehalt.

Der Gehalt des Konjugats an dem GnRH-Analogon wurde aus der Zahl der Counts pro Minute (CPM) der $^{14}$C-Markierung bestimmt. Eine Probe von 100 $\mu$l wurde zu 5 ml Aqua Sol Scintillations-Cocktail von New England Nuclear hinzugefügt und 1 min in einem Beckman-Szintillationszähler gezählt.

Es wurde gefunden, daß das Konjugat ein Verhältnis Analogon zu Träger von 7,7 Analog-Einheiten pro $10^5$ u ($10^5$ Dalton) aufwies.

Der Wirkungsgrad der Konjugation wurde zu nahezu 100 % gefunden. Statt das auf dem Analogon mit Ersatz in der Sechs-Position basierende Konjugat zu sammeln und zu vereinigen, das zwischen 3 und 6 ml eluierte, wurden aliquote Anteile von 1 ml über den gesamten Bereich der praktischen Elution der PD-10-Säule (1 bis 18 ml) aufgefangen. Jeder aliquote Anteil wurde sowohl auf seinen Protein-Gehalt als auch auf seinen Gehalt an Analogon mittels des Coomasie-Blau-Protein-Assays bzw. des $^{14}$C-CPM analysiert. Bei

geeigneter Anpassung der Skalen der graphischen Darstellungen des Protein-Gehalts und des Analogon-Gehalts in Abhängigkeit von dem Elutions-Volumen ließen sich beide graphischen Darstellungen einander überlagern. Dies zeigte an, daß das Träger-Protein und das Analogon nunmehr das gleiche Elutions-Muster aufwiesen und demnach beide Teil ein und desselben Moleküls waren.

BEISPIEL 2

Impfung mit einem Träger/GnRH-Analogon

Ein gemäß Beispiel 1 hergestelltes Konjugat basierend auf KLH und dem Analogon mit Ersatz in der Sechs-Position mit einem Konjugations-Verhältnis von 7,9 wurde zur Behandlung von 5 Balb/c-Mäusen eingesetzt. Jedem der Säuger wurde eine anfängliche intraperitoneale Injektion von 50 $\mu$g Konjugat in 200 $\mu$l Kochsalz-Losung und nachfolgend 14 Tage später eine Auffrischungs-Injektion der gleichen Formulierung und Dosis auf dem gleichen Wege verabreicht. Zum Zeitpunkt von 21 Tagen wurde den Mäusen durch Orbitalvenenpunktion Blut abgenommen, und das Serum wurde durch Assay der primären Bindung in flüssiger Phase auf Anti-GnRH-Antikörper getestet. Im einzelnen wurden 100 $\mu$l der Serum-Probe oder einer geeigneten seriellen Verdunnung über Nacht bei 4° C mit 100 $\mu$l $^{125}$I-markiertem GnRH, das 20 000 CPM pro 100 $\mu$l zeigte, in Anwesenheit von 100 $\mu$l PBS-Gel-Lösung inkubiert. Das proteingebundene, radiomarkierte GnRH wurde durch Fällung mit 1 ml kaltem Ethanol und 10 min Zentrifugieren bei 1200 g bei 4° C isoliert. Der dekantierte Niederschlag wurde in einem automatischen Micromedic-Gamma-Counter ausgewertet. Die von dem dekantierten Niederschlag erhaltenen Ergebnisse wurden als Prozentsatz der Gesamtzahl der Counts berechnet; eine ursprüngliche inkubierte Probe von 300 $\mu$l enthielt 20 000 CPM, so daß der für den dekantierten Niederschlag dieser Probe beobachtete CPM-Wert durch diese Zahl zu dividieren ist, um den Prozentsatz zu ergeben. Ein Wert von 10 % wurde willkürlich als der geeignete Bezugspunkt gewählt.

Drei der Mäuse zeigten bei einer Serum-Verdunnung von 10 : 1 wenigstens 10 % der Markierung als gebunden an, und zwei der Mäuse zeigten bei einer Serum-Verdünnung von 100 : 1 wenigstens 10 % der Markierung als gebunden an. Somit war durch dieses Konjugat eine signifikante Immunantwort provoziert worden.

BEISPIEL 3

Impfung mit Adjuvans-unterstützen Konjugaten

Der Effekt des Konjugations-Verhältnisses auf die immunologische Antwort für Adjuvans-unterstützte Formulierungen von Konjugaten GnRH-Analoga auf der Basis der GnRH-Analoga mit Ersatz in der Eins- und Sechs-Position wurde bestimmt. Diese Konjugate wurden entsprechend den Arbeitsweisen des Beispiels 1, jedoch mit Variation der Analogon-Mengen im zweiten Schritt der Konjugation, hergestellt. Die Konjugations-Verhältnisse für den Ersatz in der Eins-Stellung betrugen 3,5, 6,3 und 10,6, während die Verhältnisse für den Ersatz in der Sechs-Stellung 3,7, 7,3 und 14,6 betrugen, jeweils bezogen auf $10^5$ u ($10^5$ Dalton) des Trägers, der KLH war.

Die Konjugate wurden mit einem Adjuvans kombiniert, das dem durch Beispiel III der US-PS 3 919 411 gelehrten sehr ähnlich war. 50 $\mu$g des Konjugats in phosphatgepufferter Kochsalz-Lösung (PBS) wurden mit einer Emulsion von etwa 2 Gew.-% Carbopol 934P (einer mit Polyallylsucrose vernetzten Polyacrylsäure) in einem Gemisch von 50 : 45, bezogen auf das Volumen, aus Wasser und Baumwollsamenöl, das außerdem 2,5 Vol.-% Sorbitanmonolaurat und 2,5 Vol.-% ethoxyliertes Sorbitanmonooleat (20 mol Ethylenoxid) enthielt, kombiniert. Die Formulierung erfolgte aus 9 Vol.-Teilen Konjugat-Lösung auf 1 Vol.-Teil Emulsion.

200 $\mu$l der Adjuvans-Formulierung wurde zweimal im Abstand von 14 Tagen intraperitoneal an 30 Balb/c-Mäuse verabreicht. Jedes der sechs Konjugate (drei Konjugations-Verhältnisse für jedes der GnRH-Basis-Analoga) wurde 5 Mäusen verabreicht.

Im wesentlichen zeigten alle 30 Mäuse nach 21 Tagen eine Produktion von Antikörpern gegen natürliches GnRH. Insbesondere ergab eine 10 : 1-Verdünnung des Serums für jede Maus einen $^{125}$I-Count von wenigstens 10 % der erwarteten Gesamt-Counts für den Fall, daß das gesamte $^{125}$I-markierte GnRH bindungsfähig gewesen und durch Antikörper gebunden worden wäre. Das Serum von 16 Mäusen erreichte oder überschritt diesen Minimalwert bei einer Verdünnung von 100 : 1, und das Serum von 3 Mäusen tat dies bei einer Verdunnung von 1000 : 1. Es bestand keine deutlich erkennbare Korrelation zwischen einem der beiden GnRH-Basis-Analoga einserseits oder dem Konjugations-Verhältnis andererseits und der Antikörper-Erzeugung.

BEISPIEL 4

Effekt der Dosis bei einem Adjuvans-unterstützen Konjugat

Der Effekt von vier Dosis-Werten einer Adjuvans-unterstützten Formulierung eines mit KLH mit einem Verhältnis von 7,7 : $10^5$ u ($10^5$ Dalton) konjugierten Analogon auf der Basis eines Ersatzes in der Sechs-Position auf die Antikörper-Produktion wurde untersucht. Dosis-Werte von 10, 50, 100 und 200 $\mu$g Konjugat wurden angewandt. Fünf Balb/c-Mäuse erhielten intraperitoneal jede Dosis in einer 200$\mu$l-Injektion am Tag 0 und am Tag 14, und die Antikörper-Produktion wurde am Tag 21 untersucht. Die Formulierung und das Adjuvans waren die gleichen wie in Beispiel 3, mit der Abweichung, daß der Konjugat-Gehalt verändert wurde.

Die Antikörper-Antwort wurde in der gleichen Weise wie in den Beispielen 1 bis 3 durch die Bindung eines $^{125}$I-markierten GnRH untersucht. Keine statistisch signifikante Differenz wurde zwischen den vier Gruppen zu je fünf Mäusen beobachtet; alle vier Dosis-Werte schienen die gleiche Antwort provoziert zu haben. Alle Mäuse bis auf eine erreichten den Bezugspunkt von 10 % der Gesamtzahl der Counts bei einer Serum-Verdünnung von 10 : 1, 9 der 20 erreichten den Bezugspunkt bei einer Verdunnung von 100 : 1, und 4 der 20 erreichten den Bezugspunkt noch bei einer Verdünnung von 1000 : 1.

BEISPIEL 5

Effekt des Trägers bei Adjuvans-unterstützen Formulierungen

Fünf Konjugate wurden unter Einsatz des GnRH-Analogon mit Ersatz in der Sechs-Position und von 5 verschiedenen Trägern hergestellt. Die Konjugate wurden in der in Beispiel 3 beschriebenen Weise formuliert und verabreicht; sie wurden mit dem Adjuvans auf Polyacrylsäure-Basis kombiniert und intraperitoneal in Volumina von 200 $\mu$l und Dosen von 50 $\mu$g Konjugat an 5 Balb/c-Mäuse pro Konjugat am Tag 0 und am Tag 14 verabreicht. Die provozierte Antikörper-Produktion wurde am Tag 21 durch Bindung von $^{125}$I-markiertem GnRH ausgewertet, wie in Beispiel 2 beschrieben ist. Der Träger, das Konjugations-Verhältnis pro $10^5$ u ($10^5$ Dalton) des Trägers und der projizierte Titer für 10 % der Gesamtzahl der Counts (wie in Beispiel 3 definiert) wurden in Tabelle 1 aufgeführt. Der projizierte Titer ist extrapoliert aus den Ergebnissen bei den Verdünnungen $10^{-1}$, $10^{-2}$, $10^{-3}$ und $10^{-4}$.

## Tabelle 1

| Tier-Träger Nr. | | | Konjuga-tions-verhält-nis | Projizierter Titer für 10 % der Ge-samt-Counts |
|---|---|---|---|---|
| 1 | Schweine-Thyroglobulin | (TGB) | 8,3 | 50 |
| 2 | " | " | " | 65 |
| 3 | " | " | " | 68 |
| 4 | " | " | " | 38 |
| 5 | " | " | " | 22 |
| 6 | Rinder-Serumalbumin | (BSA) | 4,6 | 210 |
| 7 | " | " | " | 1 |
| 8 | " | " | " | 1 |
| 9 | " | " | " | 1 |
| 10 | " | " | " | 47 |
| 11 | Fissurella-Hämocyanin | (KLH) | 7,7 | 210 |
| 12 | " | " | " | 330 |
| 13 | " | " | " | 430 |
| 14 | " | " | " | 88 |
| 15 | " | " | " | 7000 |
| 16 | Pferde-gamma-Globulin | (EGG) | 15,4 | 560 |
| 17 | " | " | " | 14 |
| 18 | " | " | " | 135 |
| 19 | " | " | " | 95 |
| 20 | " | " | " | 165 |
| 21 | Tetanustoxoid | (TT) | 5,9 | 14 |
| 22 | " | " | " | 1 |
| 23 | " | " | " | 1 |
| 24 | " | " | " | 1 |
| 25 | " | " | " | 10 |

Das EGG konnte entweder mit dem KLH oder mit BSA und TGB gemeinsam durch den Mann-Whitney-Test ($p < 0,05$) statistisch gruppiert werden. BSA und TT konnten statistisch dahingehend gruppiert werden, daß sie schlechtere Ergebnisse als die drei anderen Träger lieferten.

BEISPIEL 6

10 Balb/c-Mäuse wurden gemäß dem Protokoll von Beispiel 3 mit einer Adjuvans-unterstützten Formulierung eines Konjugats eines GnRH-Analogon mit Ersatz in der Eins-Position mit KLH mit einem Konjugations-Verhältnis von 10,6 : $10^5$ u ($10^5$ Dalton) behandelt, und die Antikörper-Antwort am Tag 7 und am Tag 21 wurden entsprechend dem in Beispiel 2 beschriebenen Assay untersucht. Formulierung und Adjuvans-Unterstützung erfolgten wie in Beispiel 3. Die Ergebnisse sind in Tabelle 2 aufgeführt. Der projizierte Titer für 10 % ist extrapoliert aus den Ergebnissen bei den Verdünnungen $10^{-1}$, $10^{-2}$, $10^{-3}$ und $10^{-4}$.

Tabelle 2

| Tier | Eine Woche Projizierter Titer 10 % | Drei Wochen Projizierter Titer 10 % |
|------|------|------|
| 1 | 1 | 1550 |
| 2 | 1 | 900 |
| 3 | 1 | 10 |
| 4 | 22 | 2150 |
| 5 | 35 | 4300 |
| 6 | 1 | 2650 |
| 7 | 70 | 2800 |
| 8 | 1 | 5100 |
| 9 | 1 | 1750 |
| 10 | 1 | 5800 |

BEISPIEL 7

Impfung von Rindern

Der Effekt von zwei Impfstoff-Formulierungen auf der Basis zweier Konjugate von GnRH-Analoga mit KLH, die verschiedene Adjuvantien enthielten, wurde bestimmt. Die Konjugate wurden aus gleichen Mengen von Analoga mit Ersatz durch Cystein in den Positionen Eins und Zehn mit einem Konjugations-Verhältnis zwischen 7 und 10 gemäß den Arbeitsweisen von Beispiel 1 hergestellt. Sechs Formulierungen wurden hergestellt mit Alhydrogel, Alhydrogel mit 20, 100 und 200 µg Regressin pro Dosis, Essophor und Regressin in Öl. Die Formulierungen wurden verabreicht in Dosen von 500µg Konjugat am Tag 0 und am Tag 35. Alle Formulierungen wurden 5 Bullen und 5 Ochsen durch Injektion i.m. verabreicht. Zwei der Bullen und drei der Ochsen in jeder Gruppe waren zuvor an einem erfolglosen Versuch beteiligt gewesen und waren für die Impfung etwas stärker sensibilisiert.

Die Wirkung wurde bewertet aufgrund des Antikörper-Titers gegen GnRH und der Unterdrückung der Testosteron-Produktion. Obwohl nur einige Adjuvans-Formulierungen signifikante Titer entwickelten, waren alle Adjuvans-Formulierungen in der Lage, eine signifikante Erniedrigung des Testosteron-Spiegels bei den untersuchten Bullen zu bewirken. 7 Tiere zeigten keinen 10 %-Count der möglichen $^{125}$I-Counts bei einer Verdünnung 1 : 10, darunter zwei bereits vorher behandelte Bullen. Die Essophor-Formulierung hatte den höchsten Anteil von Nicht-Antwortern (8 von 10), darunter die zwei vorher behandelten Bullen und der eine vorher behandelte Ochse.

**Patentansprüche**

1. Mittel, das zur Stimulierung des Immunsystems eines Säugers befähigt ist, um Antikörper zu erzeugen, die eine Bindung mit dem nativen GnRH des Säugers eingehen, umfassend ein Konjugat aus
   a) einem GnRH-Molekül, in dem die in 1-, 6- oder 10-Position vorkommende Aminosäure durch Cystein ersetzt ist und worin die freie Amino- oder Carboxylgruppe des endständigen Cysteins blockiert ist,
   b) und einem an sich bekannten immunstimulierenden Protein-Träger Molekül.

2. Mittel nach Anspruch 1, dadurch gekennzeichnet, daß das Träger-Molekül ein Protein mit einem Protein-Molekulargewicht > 15 kd ist.

3. Mittel nach Anspruch 2, dadurch gekennzeichnet, daß das Konjugat unter Verwendung eines heterobifunktionellen Reagens mit einer Gruppe, die gegenüber dem Protein-Träger-Molekül reaktionsfähig und deren andere Gruppe gegenüber dem Cystein reaktionsfähig ist, gebildet wird, wobei 2-16 GnRH-Moleküle pro $10^5$ dalton Protein-Träger-Molekül eingesetzt werden.

4. Impfstoff zur Hemmung des Reproduktionsvermögens eines Säugers, umfassend
   a) ein Mittel, gemäß Anspruch 1
   und
   b) ein an sich bekanntes Adjuvans.

5. Verfahren zur Hemmung des Reproduktionsvermögens eines nichthumanen Säugers durch Stimulierung des Immunsystems desselben, um Antikörper zu erzeugen, die eine Bindung mit dem nativen GnRH des Säugers eingehen, dadurch gekennzeichnet, daß ein stimulierendes Mittel gemäß Anspruch 1 angewandt wird.

6. Verfahren zur Hemmung des Reproduktionsvermögens eines nichthumanen Säugers durch Stimulierung des Immunsystems desselben, um Antikörper zu erzeugen, die eine Bindung mit dem nativen GnRH des Säugers eingehen, dadurch gekennzeichnet, daß der Säuger mit einem Impfstoff gemäß Anspruch 5 geimpft wird.

7. Modifiziertes GnRH, das für die Erzeugung von immunstimulierenden Konjugaten mit einem Protein-Träger-Molekül geeignet ist, dadurch gekennzeichnet, daß im GnRH-Molekül, die in Position 1, 6 oder 10 vorkommende Aminosäure durch Cystein ersetzt ist.

## Claims

1. Agent capable of stimulating the immune system of a mammal to produce antibodies which enter into binding with the native GnRH of the mammal, comprising a conjugate of
   a) a GnRH molecule in which the amino acid occurring in position 1, 6 or 10 is replaced by cysteine and in which the free amino or carboxyl group of the terminal cysteine is blocked,
   b) and an immunostimulant protein carrier molecule known per se.

2. Agent according to Claim 1, characterised in that the carrier molecule is a protein with a protein molecular weight > 15 kd.

3. Agent according to Claim 2, characterised in that the conjugate is formed using a heterobifunctional reagent with one group which is able to react with the protein carrier molecule and whose other group is able to react with the cysteine, employing 2-16 GnRH molecules per $10^5$ dalton of protein carrier molecule.

4. Vaccine for inhibiting the reproductivity of a mammal, comprising
   a) an agent according to Claim 1
   and
   b) an adjuvant known per se.

5. Method for inhibiting the reproductivity of a non-human mammal by stimulating the immune system thereof to produce antibodies which enter into binding with the native GnRH of the mammal, characterised in that a stimulating agent according to Claim 1 is used.

6. Method for inhibiting the reproductivity of a non-human mammal by stimulating the immune system thereof to produce antibodies which enter into binding with the native GnRH of the mammal, characterised in that the mammal is vaccinated with a vaccine according to Claim 4.

7. Modified GnRH which is suitable for the production of immunostimulant conjugates with a protein carrier molecule, characterised in that the amino acid occurring in position 1, 6 or 10 in the GnRH molecule is replaced by cysteine.

## Revendications

1. Agent capable de stimuler le système immunitaire d un mammifère afin de produire des anticorps qui réalisent une liaison avec la GnRH native du mammifère, comprenant un conjugué de:

   a) une molécule de GnRH dans laquelle les acides aminés existant en position 1, 6 ou 10 sont remplacés par la cystéine et dans laquelles les groupes amino- ou carboxyle libres de la cystéine en position terminale sont bloqués,

   b) et une molécule de protéine porteuse connue en soi pour avoir un effet de stimulation de l'immunité.

2. Agent selon la revendication 1, caractérisé en ce que la molécule porteuse est une protéine avec un poids moléculaire de la protéine > 15 kd.

3. Agent selon la revendication 2, caractérisé en ce que le conjugué est formé en utilisant un réactif hétérobifonctionnel avec un groupe qui est réactif vis-à-vis de la molécule de protéine porteuse et dont l'autre groupe est réactif vis-à-vis de la cystéine, en utilisant 2-16 molécules de GnRH pour $10^5$ Dalton de molécule de protéine porteuse.

4. Substance d'inoculation pour inhiber la fertilité d'un mammifère, comprenant:

   a) un agent selon la revendication 1

   et

   b) un adjuvant connu en soi.

5. Procédé pour inhiber la fertilité d'un mammifère non-humain, par stimulation du système immunitaire de celui-ci, afin de produire des anticorps qui donnent lieu à une liaison avec la GnRH native du mammifère, caractérisé en ce que l'on utilise un agent stimulant selon la revendication 1.

6. Procédé pour inhiber la fertilité d'un mammifère non-humain par stimulation du système immunitaire de celui-ci, afin de produire des anticorps qui donnent lieu à une liaison avec la GnRH native du mammifère, caractérisé en ce que le mammifère est traité par inoculation avec une substance d'inoculation selon la revendication 5.

7. GnRH modifiée, qui convient pour la formation de conjugués stimulant l'immunité, avec une molécule de protéine porteuse, caractérisé en ce que l'acide aminé existant en position 1, 6 ou 10 dans la molécule de GnRH est remplacé par de la cystéine.